# EUROPÄISCHE PATENTSCHRIFT

(11) **EP 2 451 772 B1**
(45) Veröffentlichungstag und Bekanntmachung des Hinweises auf die Patenterteilung: **24.07.2013**
(21) Anmeldenummer: 10726977.1
(22) Anmeldetag: 28.06.2010
(51) Int. Cl.: C07C 253/30, C07C 255/23

(54) **VERFAHREN ZUR HERSTELLUNG VON CYANACRYLSÄUREESTERN IN GEGENWART VON ÜBERGANGSMETALL-KATALYSATOREN**
METHOD FOR PRODUCING CYANOACRYLATE ESTERS IN THE PRESENCE OF TRANSITION METAL CATALYSTS
PROCÉDÉ DE PRODUCTION D'ESTERS D'ACIDE CYANOACRYLIQUE EN PRÉSENCE DE CATALYSEURS À BASE DE MÉTAUX DE TRANSITION

(30) Priorität: 08.07.2009 DE 102009027545
(43) Veröffentlichungstag der Anmeldung: 16.05.2012
(73) Patentinhaber: Henkel AG & Co. KGaA, 40589 Düsseldorf (DE)
(72) Erfinder: FRIESE, Carsten, 40217 Düsseldorf (DE); KIRSCHNING, Andreas, 29221 Celle (DE); COUTABLE, Ludovic, 89077 Ulm (DE)
(86) Internationale Anmeldenummer: PCT/EP2010/059155
(87) Internationale Veröffentlichungsnummer: WO 2011/003768

(56) Entgegenhaltungen:
- EP-A1- 0 764 148
- WO-A1-02/072535
- SU-A1- 726 086

## Beschreibung

Die vorliegende Erfindung betrifft ein Verfahren zur Herstellung von Cyanacrylsäureestern. Das Verfahren beruht im Wesentlichen auf einer Umesterungsreaktion, wobei die Umesterungsreaktion in Gegenwart mindestens eines Übergangsmetall-Katalysators durchgeführt wird, der durch Umsetzung mindestens eines Hydroxylgruppen-haltigen Trägermaterials mit mindestens einem Übergangsmetallalkoxid gebildet wird.

Polymerisierbare Klebstoffzusammensetzungen auf Basis von Cyanacrylsäureestern haben aufgrund ihrer einfachen Anwendbarkeit sowie der hohen Aushärtegeschwindigkeit und Festigkeit der resultierenden Klebeverbindung eine breite Verwendung sowohl in industriellen als auch in medizinischen Anwendungen gefunden. Es ist bekannt, dass monomere Formen von Cyanacrylaten extrem reaktiv sind und in der Gegenwart von selbst kleinsten Mengen eines Polymerisationsinitiators, einschließlich der in der Luft enthaltenen oder an Oberflächen vorhandenen Feuchtigkeit schnell polymerisieren. Dabei erfolgt die Initiation der Polymerisation durch Anionen, freie Radikale, Zwitterionen oder Ionenpaare. Wenn die Polymerisation einmal gestartet worden ist, kann die Aushärtegeschwindigkeit sehr groß sein. Polymerisierbare Klebstoffzusammensetzungen auf Basis von Cyanacrylsäureester haben sich daher beispielsweise beim Verbinden von Kunststoffen, Kautschuken, Glas, Metallen, Holz und auch biologischen Geweben als attraktive Lösungen erwiesen.

Wie im US Patent 6,667,031 offenbart, beruht die herkömmliche Synthese von Mono-Cyanacrylsäureestern auf einer Knoevennagel-Kondensation, in der Cyanacetat mit Formaldehyd umgesetzt wird. Das dabei entstandene Präpolymers wird bei hohen Temperaturen von 150 bis über 200°C durch thermisches Cracken depolymerisiert, wobei die Abtrennung der gebildeten Monomere von der Reaktionslösung in der Regel durch Destillation erfolgt. Die thermische Depolymerisation gelingt nur, wenn dieser Prozess in der Gegenwart von Stabilisatoren oder Mischungen von Stabilisatoren abläuft, die sowohl eine radikalische als auch eine anionische Repolymerisation der gebildeten Monomere unter den beschriebenen Reaktionsbedingungen verhindern können.
Stabilisatoren, die eine anionische Repolymerisation verhindern, sind in der Regel, aber nicht ausschließlich, Lewissäuren, wie beispielsweise Schwefeldioxid, Stickstoffmonoxid oder Bortrifluorid oder anorganische oder organische Brønstedtsäuren, wie beispielsweise Schwefelsäure, Phosphorsäure oder organische Sulfonsäuren.

Oft führt die hohe Konzentration der in dem Prozess verwendeten Stabilisatoren dazu, dass bei der destillativen Abtrennung des Monomers von der Reaktionslösung ein Teil des Stabilisators verschleppt wird, wodurch eine hohe Restkonzentration des Stabilisators im destillierten Cyanacrylatmonomer entstehen kann. Insbesondere bei sterisch anspruchsvollen Cyanacrylsäureestern kann auf diese Weise eine unerwünschte Überstabilisierung des Monomers entstehen, wodurch deren Polymerisationsgeschwindigkeit reduziert wird.

Auch die Herstellung von Biscyanacrylaten ist seit langem bekannt und kann folgendermaßen erfolgen:

Analog zur oben beschriebenen Knoevenagel-Kondensation werden Formaldehyd und Biscyanacetate umgesetzt. Dabei entsteht ein vernetztes Polymer, welches kaum noch thermisch depolymerisiert werden kann.

Weiterhin kann die Herstellung von Biscyanacrylaten in einer Retro-Diels-Alder-Reaktion erfolgen. Wie beispielsweise im US-Patent 3,975,422 beschrieben, wird zunächst ein monofunktionelles Cyanacrylat mit Dienen blockiert. Das blockierte monofunktionelle Cyanacrylat wird zur freien Säure verseift. Aus dem entsprechenden Säurechlorid wird mit einem Diol dann der Ester hergestellt. Nach dem Austausch des Biscyanacrylates gegen Maleinsäureanhydrid wird schließlich nach wiederholtem Umkristallisieren aus Benzol das reine Biscyanoacrylat erhalten. Dieser Herstellungsweg umfasst fünf Stufen und ist daher unwirtschaftlich.

Eine alternative Möglichkeit zur Herstellung von Biscyanacrylaten wird in der europäischen Patentschrift EP 0764148 B1 gelehrt.
Die Biscyanoacrylate werden durch Reaktion von 2-Cyanacrylsäure oder deren Alkylester mit Diolen erhalten, wobei die Reaktion vorzugsweise in Gegenwart von Sulfonsäuren als Katalysator in Lösung durchgeführt wird. Die Biscyanacrylate werden anschließend durch fraktionierte Kristallisation isoliert.

Darüber hinaus wird in der SU 726086 A1 ein Umesterungsverfahren gelehrt, beim dem 2-Cyanacrylsäureester in Gegenwart von Schwefelsäure-, Sulfonsäure- oder Zinkchlorid-Katalysatoren hergestellt werden.

Die WO 2002/072535 A1 offenbart ein Umesterungsverfahren von 2-Cyanacrylsäureestern in Gegenwart von Trisulfonsäure als Katalysator.

Als nachteilig an diesem Verfahren hat sich erwiesen, dass relativ hohe Mengen an Sulfonsäuren eingesetzt werden müssen, um eine effektive Veresterungs- oder Umesterungsreaktion zu erreichen. Eine vollständige Abtrennung der Sulfonsäuren vom erhaltenen Produkt ist oft schwierig, wodurch gerade bei sterisch aufwendigen Biscyanacrylaten eine deutliche Reduktion der Polymerisationsgeschwindigkeit des jeweiligen Monomers zu beobachten ist, da die genannten Sulfonsäuren als anionische Polymerisationsinhibitoren wirken.

Neben den beschriebenen homogen-katalysierten Verfahren sind im Stand der Technik auch Umesterungsverfahren bekannt, die in Gegenwart heterogener Katalysatoren ablaufen.

X. Ma, S. Wang, J. Gong, X. Yang und G. Xu (Journal of Molecular Catalysis A: Chemical 222 (2004) 183-187) beschreiben beispielsweise die Herstellung von TiO₂-, TiO₂/SiO₂-, TiO₂/Al₂O₃- und TiO₂/MgO-basierter Katalysatoren und deren erfolgreichen Einsatz als heterogene Katalysatorsysteme in Umesterungsreaktionen des Dimethyloxalats.

D. Srinivas, R. Srivastava und P. Ratnasamy (Catalysis Today 96 (2004) 127 - 133) beschreiben die Herstellung ähnlicher Katalysatorsysteme und deren erfolgreichen Einsatz als heterogene Katalysatorsysteme in Umesterungsreaktionen des Ethylacetonats, Dimethylmalonats und zyklischer Propylencarbonate.

In der EP 0574632 B1 wird ein Verfahren zur Herstellung von Carbonsäureestern gelehrt, indem ein Katalysator verwendet wird, der durch Umsetzung vorgefertigter Übergangsmetalloligomere mit einem festen Substrat, das an seiner Oberfläche Hydroxylgruppen aufweist, erhalten wird.

Keiner der genannten Schriften offenbart den Einsatz der heterogenen Katalysatoren in Umesterungsreaktionen von Cyanacrylsäureestern.

Ziel der vorliegenden Erfindung war die Entwicklung eines alternativen Verfahrens zur Herstellung von Cyanacrylsäureestern, das es erlaubt die genannten Ester in hoher Reinheit mit einer möglichst geringen Restkonzentration an anionischen Polymerisationsinhibitoren herzustellen.

Die erfindungsgemäße Lösung ist den Ansprüchen zu entnehmen. Sie besteht im Wesentlichen in der Umesterung von Cyanacrylsäureestern mit einwertigen und/oder mehrwertigen Alkoholen in Gegenwart mindestens eines Übergangsmetall-Katalysators, der durch Umsetzung mindestens eines Hydroxylgruppen-haltigen Trägermaterials mit mindestens einem Übergangsmetallalkoxid gebildet wird.

Der mindestens eine Übergangsmetall-Katalysator erlaubt es die Umesterungsreaktion des erfindungsgemäßen Verfahrens auch ohne die Zugabe von Brønstedtsäuren mit hohen Ausbeuten durchzuführen, wodurch Umesterungsprodukte von hoher Reinheit und hoher Reaktivität erhalten werden, die einen äußerst geringe Restkonzentration an anionischen Polymerisationsinhibitoren aufweisen.

Gegenstand der vorliegenden Erfindung ist daher ein Verfahren zur Herstellung mindestens eines Cyanacrylsäureesters der allgemeinen Formel (I), 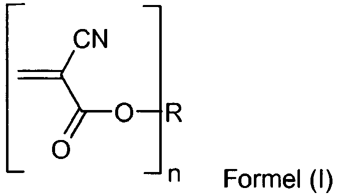 umfassend die Schritte:
a) Umesterung mindestens eines 2-Cyanacrylsäureesters der allgemeinen Formel (II), 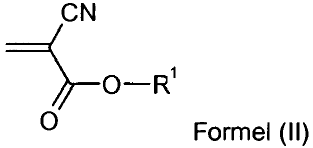 wobei R¹ ein verzweigter oder unverzweigter Alkylrest mit 1 bis 6 C-Atomen ist, mit mindestens einem Alkohol der allgemeinen Formel (III),

   [H-O-]ₙR Formel (III)

   in Gegenwart mindestens eines Übergangsmetall-Katalysators, der durch Umsetzung mindestens eines Hydroxylgruppen-haltigen Trägermaterials mit mindestens einem Übergangsmetallalkoxid gebildet wird und
b) Isolierung des in Schritt a) erhaltenen Cyanacrylsäureesters der allgemeinen Formel (I),
wobei n eine ganze Zahl von 1 bis 6 ist und R ein n-valenter Rest ist, der 1 bis 100 C-Atomen umfasst.

Da es sich bei dem erfindungsgemäßen Verfahren um eine Umesterungsreaktion handelt, können die Reste R und R¹ nicht identisch sein. Insbesondere ist es bevorzugt, dass Rest R mindestens ein C-Atom, insbesondere mindestens zwei C-Atome mehr als Rest R¹ aufweist.

Als Übergangsmetall-Katalysator im Sinne der vorliegenden Erfindung sind alle Verbindungen geeignet, die durch Umsetzung mindestens eines Hydroxylgruppen-haltigen Trägermaterials mit mindestens einem Übergangsmetallalkoxid gebildet werden und die eine katalytische Aktivität in der im erfindungsgemäßen Verfahren durchgeführten Umesterungsreaktion aufweisen.

Der Begriff "Übergangsmetall" kennzeichnet im Sinn der Erfindung ein metallisches Element, welches aus den Gruppen Ib, IIb, IIIa (einschließlich der Lanthanoide), IVa, Va VIa, VIIa und VIII des Periodensystem der Elemente, veröffentlicht im Berblatt des Bulletin de la Societe Chimique de France Nr. 1 (Januar 1966), gewählt ist. Anders formuliert handelt es sich um ein Element, dessen Ordnungszahl zwischen einschließlich 21 und 30, zwischen einschließlich 39 und 48 oder zwischen einschließlich 57 und 80 liegt.

Unter einem n-valenten Rest R ist im Sinne der vorliegenden Erfindung ein n-valentes organisches Radikal mit 1 bis 100 C-Atomen zu verstehen, das ausgehend vom Cyanacrylsäureester der allgemeinen Formel (I) oder Alkohol der allgemeinen Formel (III) formal durch die homolytische Spaltung von n Kohlenstoff-Sauerstoff-Bindungen gebildet wird.

Der Begriff des n-valenten Rests soll im Folgenden exemplarisch an dem Beispiel des dreiwertigen Alkohols Trimethylolpropan (TMP; n = 3) näher erläutert werden:

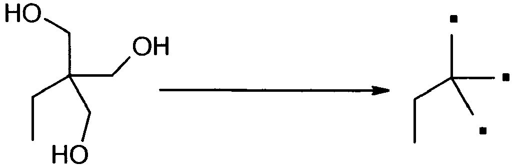

Durch die formale homolytische Spaltung der drei C-OH-Bindungen im TMP-Molekül entsteht im Sinne der vorliegenden Erfindung ein trivalenter organischer Rest.

Mit dem erfindungsgemäßen Verfahren lassen sich in Abhängigkeit vom verwendeten Alkohol sowohl Mono-Cyanacrylsäureester als auch höhere Cyanacrylsäureester herstellen.

In einer bevorzugten Ausführungsform des erfindungsgemäßen Verfahrens wird der Cyanacrylsäureester der allgemeinen Formel (I) daher aus Mono-cyanacrylsäureestern der allgemeinen Formel (la), 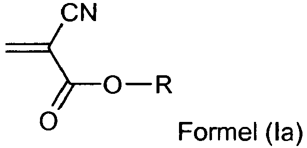 und/oder aus Biscyanacrylsäureestern der allgemeinen Formel (Ib), 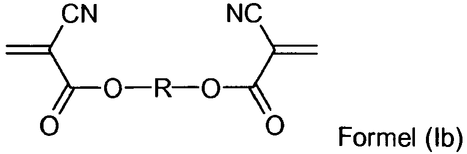 und/oder aus Triscyanacrylsäureestern der allgemeinen Formel (Ic) 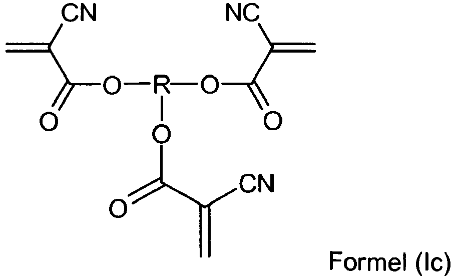 ausgewählt, wobei die Reste R wie oben definiert sind.

Insbesondere eignet sich das erfindungsgemäße Verfahren zur Herstellung von Biscyanacrylsäureestern der allgemeinen Formel (Ib) und/oder Triscyanacrylsäureestern der allgemeinen Formel (Ic). Aufgrund der hohen Reaktivität des verwendeten Übergangsmetall-Katalysators lassen sich die vorgenannten Cyanacrylsäureester in sehr hohen Reinheiten darstellen. Gleichzeitig weisen die genannten Moleküle eine sehr hohe Reaktivität in den entsprechenden Polymerisationsreaktionen auf, da im erfindungsgemäßen Umesterungsverfahren vollständig oder nahezu vollständig auf die Verwendung von Brønstedtsäuren als Umesterungskatalysatoren verzichtet wird.

In einer bevorzugten Ausführungsform des erfindungsgemäßen Verfahrens umfasst der Rest R in Formel (I), Formel (la), Formel (Ib), Formel (Ic) und/oder Formel (III) eine C₃ bis C₁₀₀-Kette, vorzugsweise eine C₅ bis C₇₀-Kette und insbesondere eine C₁₀ bis C₅₀-Kette, die durch mindestens ein Sauerstoffatom unterbrochen ist. Der Rest R kann dabei linear, verzweigt oder zyklisch angeordnet sein.
Insbesondere sind Reste R bevorzugt, die mindestens eine Polyethylenglykol- und/oder mindestens eine Polypropylenglykoleinheit aufweisen.

In einer weiteren Ausführungsform des erfindungsgemäßen Verfahrens umfasst der Rest R in Formel (I), Formel (la), Formel (Ib), Formel (Ic) und/oder Formel (III) 3 bis 18, insbesondere 4, 8, 10 und/oder 12 direkt miteinander verbundene C-Atome. Der Rest R kann dabei ebenfalls linear, verzweigt oder zyklisch angeordnet sein.

Der Rest R kann weiterhin eine aromatische Gruppe enthalten oder neben Wasserstoff- und Kohlenstoffatomen auch noch mindestens ein Heteroatom, beispielsweise ausgewählt aus Halogen-, Sauerstoff- und/oder Stickstoffatomen, umfassen.

Bei den Alkoholen der allgemeinen Formel (III)

[H-O-]ₙR Formel (III)

handelt es sich um einwertige bis sechswertige Alkohole (n = 1 - 6), wobei ein-, zwei- und/oder dreiwertige Alkohole bevorzugt sind. Mit Hilfe dieser Alkohole sind die oben gezeigten Mono-cyanacrylsäureester der allgemeinen Formel (la), die Biscyanacrylsäureester der allgemeinen Formel (Ib) und die Triscyanacrylsäureester der allgemeinen Formel (Ic) leicht darstellbar.
Die im erfindungsgemäßen Verfahren verwendeten Alkohole sind vorzugsweise primäre oder sekundäre Alkohole, wobei primäre Alkohole bevorzugt sind, da diese eine höhere Reaktivität in der Umesterungsreaktion aufweisen. Generell können auch beliebige Mischungen unterschiedlicher Alkohole verwendet werden.

Brauchbare Alkohole können beispielsweise ausgewählt werden aus Ethanol, Chlorethanol, Cyanoethanol, n-Propanol, sec-Propano1,-n-Butanol, tert-Butano1, Isoamylalkohol, n-Hexanol, 2-Ethylhexanol, n-Octanol, n-Decanol, Isodecanol, Cyclohexanol, Benzylalkohol, ortho-, meta- und para-Methoxybenzylalkohol, Ethylenglycol, 1,2-Propylenglycol, 1,3-Propylenglycol, 1,2-Butandiol, 1,3-Butandiol, 1,4-Butandiol, 1,4- Pentandiol, 3-Methyl-1,5-pentandiol,2-,3-Dimethyl-2,3-butandiol,Phenylethylalkohol, Triphenylethylalkohol, Trimethylolpropan, Mannitol, Sorbitol, Glycerin, Pentaerythritol, 1,4-Cyclohexandimethanol, Xylenol, Bisphenole, Diethylenglycol, Triethylenglycol, Polyoxyethylen-oder Polyoxypropylenglycole vorzugsweise mit einem Molekulargewicht bis etwa 4.000, Diethylenglycolmonomethylether, Diethylenglycolmonoethylether, Triethylenglycolmonomethylether, Butoxyethanol, Butylenglycolmonobutylether, Dipentaerythritol, Tetrapentaerythritol, Diglycerin, Triglycerin und dergleichen.

Der im erfindungsgemäßen Verfahren eingesetzte Alkohol der allgemeinen Formel (III) wird in einer bevorzugten Ausführungsform der vorliegenden Erfindung auch aus Verbindungen der allgemeinen Formel (IIIa)

R ²COO(CH₂CH₂ O)ₘH Formel (IIIa)

ausgewählt, in der R²CO für einen linearen oder verzweigten Acylrest mit 12 bis 22 Kohlenstoffatomen und m für Zahlen von 5 bis 30 und vorzugsweise 15 bis 25 steht. Typische Beispiele sind Anlagerungsprodukte von Ethylenoxid an Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen oder bei der Reduktion von Aldehyden aus der Roelen'schen Oxosynthese anfallen. Vorzugsweise werden Anlagerungsprodukte von Ethylenoxid an Fettsäuren mit 16 bis 18 Kohlenstoffatomen eingesetzt.

Gemäß dem erfindungsgemäßen Verfahren werden die Alkohole der Formel (III) mit mindestens einem 2-Cyanacrylsäureester der allgemeinen Formel (II) umgesetzt, 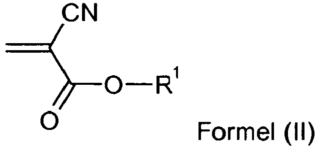 wobei R¹ein verzweigter oder unverzweigter Alkylrest mit 1 bis 6 C-Atomen ist.

Methyl-2-cyanacrylat oder Ethyl-2-cyanacrylat sind im Sinne der vorliegenden Erfindung bevorzugte 2-Cyanacrylsäureester, da der in der Umesterungsreaktion entstehende Alkohol Methanol oder Ethanol leicht entfernt werden kann. Es können ebenfalls Mischungen verschiedener 2-Cyanacrylsäureester eingesetzt werden, wobei Mischungen aus Methyl-2-cyanacrylat oder Ethyl-2-cyanacrylat bevorzugt sind.

Der mindestens eine 2-Cyanoacrylsäureester der allgemeinen Formel (II) wird im erfindungsgemäßen Verfahren vorzugsweise im Überschuss eingesetzt. Das molare Verhältnis von 2-Cyanoacrylsäureester zum Alkohol der allgemeinen Formel (III) reicht dabei von 20:1 bis 1:1, vorzugsweise von 10: 1 bis 1,5:1.

Die Umesterung (Schritt a) des erfindungsgemäßen Verfahrens wird in Gegenwart mindestens eines Übergangsmetall-Katalysators durchgeführt, wobei der genannte Übergangsmetall-Katalysator durch Umsetzung mindestens eines Hydroxylgruppen-haltigen Trägermaterials mit mindestens einem Übergangsmetallalkoxid gebildet wird, das vorzugsweise ausgewählt wird aus Übergangsmetallalkoxiden der allgemeinen Formel (IV),

M(OR³)ₐ₋ᵤR^{4u} Formel (IV)

wobei a für 3, 4 oder 5 steht, u entweder 0, 1 oder 2 ist, M für ein Übergangsmetall steht, R³ eine lineare oder verzweigte gegebenenfalls substituierte C1-20 Alkylgruppe oder eine gegebenenfalls substituierte C6-12 Arylgruppe ist und R⁴ für eine lineare oder verzweigte gegebenenfalls substituierte C1-4 Alkylgruppe steht.

Der im erfindungsgemäßen Verfahren verwendete Übergangsmetall-Katalysator zeichnet sich vorzugsweise dadurch aus, dass er im Reaktionsgemisch im Wesentlichen unlöslich ist.

Unter dem Begriff "Reaktionsgemisch" ist eine Mischung zu verstehen, die mindestens einen Cyanacrylsäureester der allgemeinen Formel (I) und mindestens einen Alkohol der allgemeinen Formel (III) und ggf. weitere Lösungsmittel, wie beispielsweise organische Lösungsmittel, umfasst.

Unter dem Begriff "im Wesentlichen unlöslich" ist im Sinne der Erfindung vorzugsweise zu verstehen, dass unter den jeweils gewählten Reaktionsbedingungen weniger als 20 mol-% , vorzugsweise weniger als 10 mol-%, besonders bevorzugt weniger als 5 mol-% und insbesondere weniger als 1 mol-% aller Übergangsmetallatome des ursprünglich verwendeten Übergangsmetall-Katalysators in der Reaktionsmischung nachgewiesen werden können. Der Nachweis kann beispielsweise durch Atomemissionsspektroskopie (AES) erfolgen.

Bei der Umesterungsreaktion der vorliegenden Erfindung handelt es sich um ein heterogen-katalysiertes Verfahren. Ein solches Verfahren ist vorteilhaft, da der Übergangsmetall-Katalysator leicht von der Reaktionsmischung abgetrennt werden kann und das Verfahrensprodukt nur eine äußerst geringe Restkonzentration des genannten Katalysators aufweist.

In einer speziellen Ausführungsform wird mindestens ein Übergangsmetall im Übergangsmetall-Katalysator und insbesondere das Übergangsmetall M in Formel (IV) ausgewählt aus Titan, Zirkonium, Hafnium, Eisen, Zink oder Vanadium, wobei Titan besonders bevorzugt ist.

Die vorgenannten Alkyl- und/oder Arylgruppen können ein oder mehrere Halogenatome und/oder Heteroatome als Substituenten tragen, wobei die Heteroatome Bestandteil einer funktionellen Gruppe sind, die beispielsweise ausgewählt wird aus primären und sekundären Aminen, Amiden, Urethanen, Alkoholen, Ethern, Estern, Thiolen, Thioethern und Sulfonen

Als Alkoxygruppe OR² mit 1 bis 20 C-Atomen sind insbesondere Methoxy-, Ethoxy-, n-Propoxy-, Isopropoxy-, n-Butoxy-, Isobutoxy-, tert-Butoxy-, 2-Ethylhexyloxy-, n-Decyloxy-, Tridecyloxy- und Phenoxygruppen bevorzugt, wobei als Alkoxygruppe OR² insbesondere Alyklgruppen mit 1 bis 4 C-Atomen, vorzugsweise mit 2, 3 oder 4 C-Atomen verwendet werden können.

Für den Fall, dass u den Wert 1 hat, wird R³ vorzugsweise ausgewählt aus Methyl-, Ethyl-, Propyl- und iso-Propylgruppen.

Das Übergangsmetallalkoxid der vorliegenden Erfindung ist insbesondere ein Titantetraalkoxid, wie etwa Tetra-n-butyltitanat, Tetra-t-butyltitanat, Tetra-n-propyltitanat, Tetra-isopropyltitanat, Tetraphenyltitanat, Tetracyclohexyltitanat und Tetrabenzyltitanat oder eine Trialkoxy-monoalkyl Titanverbindung.

Weiterhin kann das Übergangsmetallalkoxid der allgemeinen Formel (IV) vor und/oder während der Umsetzung mit dem Hydroxylgruppen-haltigen Trägermaterial durch Teilhydrolyse in ein Übergangsmetalloligomer überführt werden.

Unter einem Übergangsmetalloligomer ist im Sinne der vorliegenden Erfindung insbesondere eine Verbindungen zu verstehen, die 10 bis 10000 Übergangsmetallatome umfasst, wobei die einzelnen Übergangsmetallatome jeweils durch verbrückende Sauerstoffatome miteinander verknüpft sind.

Die Wassermenge kann in Abhängigkeit vom Ausmaß des erwünschten Oligomerisierungsgrads in einem weiten Bereich schwanken. Ein Mol Wasser ist für jedes Mol Sauerstoffbrücke, die gebildet wird, erforderlich. Es werden im Rahmen der Teilhydrolyse sowohl geradkettige als auch verzweigte Oligomere gebildet. Die Teilhydrolyse kann in jedem organischen Lösungsmittel durchgeführt werden, in welchem das Übergangsmetallalkoxid löslich und stabil ist. Sie wird jedoch typischerweise in dem Alkohol ausgeführt, der jenem des Übergangsmetallalkoxids entspricht. Gleichermaßen kann das Wasser rein oder verdünnt sein. Oft wird es mit dem Lösungsmittel verdünnt, das verwendet wird, um das Übergangsmetallalkoxid aufzulösen. Das Wasser oder die Wasserlösung wird gewöhnlicherweise tropfenweise zugegeben, wobei die Temperatur, der Druck und die Zugabegeschwindigkeit der jeweiligen Reaktivität des Übergangsmetallalkoxids anzupassen sind. Nach der Reaktion braucht das Lösungsmittel nicht zwingend entfernt zu werden. Für die Umsetzung mit dem Hydroxylgruppen-haltigem Trägermaterial kann es zweckmäßig sein, dass Lösungsmittel zu entfernen und durch ein anderes Lösungsmittel zu ersetzen.

In einer alternativen Ausführungsform erfolgt die Umsetzung des Übergangsmetallalkoxids, insbesondere des Übergangsmetallalkoxids der allgemeinen Formel (IV) mit dem Hydroxylgruppen-haltigen Trägermaterial unter wasserfreien Bedingungen. Auf diese Weise wird die Tendenz zur Oligomerisierung minimiert, wodurch andere Übergangsmetall-Katalysatoren mit anderen Reaktivitäten erhalten werden.

Das Hydroxylgruppen-haltige Trägermaterial unterliegt in seiner Struktur und seiner chemischen Zusammensetzung im Sinne der vorliegenden Erfindung keinen speziellen Limitierungen, sofern es eine Anbindung (Immobilisierung), insbesondere die kovalente Anbindung mindestens eines geeigneten Übergangsmetallalkoxids erlaubt.

Üblicherweise werden als Hydroxylgruppen-haltige Trägermaterialien solche verwendet, die an der Oberfläche eine Vielzahl von Hydroxylgruppen aufweisen.
Das Hydroxylgruppen-haltige Trägermaterial kann dabei entweder natürlich oder synthetisch sein. Beispiele sind Kohlenhydrat-basierte Polymere, Cyclodextrine, Aluminiumoxid, Siliciumdioxid, Quarzstaub, Silicagel, Tone (wie z.B. Kaolinit, Montmorillonit, Vermiculit, Chlorit und Glimmer-Typen), Zeolithe, Zirkoniumoxid, Titanoxid, Thoriumoxid, Magnesiumoxid, Aluminate, Kohleschwarz, synthetische anorganische Oxide von Silicium, Magnesium, Aluminium, Zink, und ihre Gemische und dergleichen geeignet.
Das Hydroxylgruppen-haltige Trägermaterial ist vorzugsweise ausgewählt aus Siliciumdioxid, Aluminiumoxid und Gemischen davon. Zusätzlich sind auch organische Polymere mit Hydroxylguppen brauchbar. Oxide von Silicium und Aluminium sind bevorzugte Hydroxylgruppen-haltige Trägermaterialien.

Geeignete Verfahren zur Herstellung der Übergangsmetall-Katalysatoren der vorliegenden Erfindung können beispielsweise den vorgenannten Veröffentlichungen von X. Ma, S. Wang, J. Gong, X. Yang und G. Xu (Journal of Molecular Catalysis A: Chemical 222 (2004) 183 - 187) und D. Srinivas, R. Srivastava und P. Ratnasamy (Catalysis Today 96 (2004) 127 - 133) entnommen werden.

In einer speziellen Ausführungsform wird der Übergangsmetall-Katalysator der vorliegenden Erfindung vor dem Einsatz im erfindungsgemäßen Verfahren calciniert, d.h. über einen gewissen Zeitraum hinweg in Gegenwart von Sauerstoff einer erhöhten Temperatur ausgesetzt.

Brauchbare Übergangsmetall-Katalysatoren können daher auch durch Umsetzung mindestens eines Hydroxylgruppen-haltigen Trägermaterials mit mindestens einem Übergangsmetallalkoxid erhalten werden, wobei das Umsetzungsprodukt anschließend in Gegenwart von Sauerstoff Temperaturen von 150°C bis 800°C ausgesetzt wird. Bevorzugte Temperaturen liegen zwischen 300°C und 700°C, insbesondere zwischen 400°C und 600°C.

Geeignete Sauerstoffquellen sind insbesondere Mischungen verschiedener Gase, wobei der Sauerstoffgehalt mindestens 10 Gew.-% betragen sollte. Insbesondere kann die Calcinierung in Gegenwart von Luft durchgeführt werden.

Durch die Calcinierung wird die chemische Struktur des Übergangsmetall-Katalysators entscheidend verändert, wodurch in vielen Fällen die katalytische Aktivität gesteigert wird oder eine Anpassung der katalytischen Aktivität an die speziellen Anforderungen einer bestimmten Umesterungsreaktion gelingt.

Selbstverständlich können im Rahmen der vorliegenden Erfindung beliebige Mischungen verschiedener Übergangsmetall-Katalysatoren verwendet werden.

Der mindestens eine Übergangsmetall-Katalysator wird in Abhängigkeit von der Reaktivität der einzelnen Reaktanten und den gewählten Reaktionsbedingungen vorzugsweise in Mengen von 0,01 bis 10 Gew.-%, besonders bevorzugt von 0,1 bis 5 Gew.-% und insbesondere von 1 bis 3 Gew.-%, jeweils bezogen auf die Gesamtmenge des 2-Cyanacrylsäureesters der allgemeinen Formel (II), eingesetzt.

Das erfindungsgemäße Verfahren wird vorzugsweise in Gegenwart mindestens eines radikalischen Polymerisationsinhibitors durchgeführt. Der radikalische Polymerisationsinhibitor verhindert eine vorzeitige Polymerisation der eingesetzten Cyanacrylsäureester. Die benötigte Menge kann von einem Fachmann leicht bestimmt werden. Geeignete radikalische Polymerisationsinhibitoren sind beispielsweise Phenolverbindungen, wie etwa Hydrochinon, Hydrochinonmonomethylether, butyliertes Hydroxyanisol (BHA), 2,6-Di-tert-butyl-4-methylphenol (BHT), t-Butylcatechinon, Pyrocatechin und p-Methoxyphenol. Mischungen der genannten radikalischen Polymerisationsinhibitoren können ebenfalls verwendet werden. Ein besonders bevorzugter radikalischer Polymerisationsinhibitor ist im Sinne der vorliegenden Erfindung butyliertes Hydroxyanisol (BHA) und/oder Hydrochinonmonomethylether.

Weiterhin wird das erfindungsgemäße Verfahren vorzugsweise in Gegenwart einer bei 22°C gasförmigen Säure, insbesondere einer Lewissäure durchgeführt, wobei die Säure beispielsweise ausgewählt wird aus Schwefeldioxid und/oder Bortrifluorid. Die gennanten Säuren stabilisieren dabei die jeweiligen Cyanacrylsäureester.

Die Umesterungsreaktion des vorliegenden Verfahrens kann bei Normaldruck, aber auch bei reduziertem oder Überdruck von 0,001 bis 200 bar durchgeführt werden. Der bei der Umesterung abgespaltene Alkohol wird bevorzugt kontinuierlich oder nach Beendigung der Reaktion abdestilliert, wobei die Umsetzung in einem geeigneten Lösungsmittel, wie etwa Toluol, Xylol oder Toluol/Xylol-Mischungen durchgeführt werden kann. Bevorzugt wird das erfindungsgemäße Verfahren mit Hilfe einer Destillationsvorrichtung mit hoher Trennleistung durchgeführt.

In einer speziellen Ausführungsform wird die Umesterungsreaktion (Schritt a des erfindungsgemäßen Verfahrens) unter Erwärmen des Reaktionsgemisches in einem Reaktor in Gegenwart mindestens eines Übergangsmetall-Katalysators der vorliegenden Erfindung durchgeführt, wobei das Reaktionsgemisch mindestens einen Cyanacrylsäureester der allgemeinen Formel (II) und mindestens einen Alkohol der allgemeinen Formel (III) als Reaktanten umfasst und in Kontakt mit einem durch elektromagnetische Induktion erwärmbaren festen Heizmedium steht, das sich innerhalb des Reaktors befindet und das durch elektromagnetische Induktion mit Hilfe eines Induktors erwärmt wird.

Vorteilhaft ist, dass die thermische Energie zur Durchführung der Umesterungsreaktion nicht durch Oberflächen wie beispielsweise die Reaktorwände, Heizschlangen, Wärmetauscherplatten oder ähnliches in das Reaktionsgemisch eingebracht wird, sondern direkt im Volumen des Reaktors erzeugt wird. Das Verhältnis von erwärmter Oberfläche zu Volumen des Reaktionsgemischs kann dabei wesentlich größer werden als bei einer Heizung über Wärme übertragende Oberflächen. Zusätzlich wird der Wirkungsgrad von elektrischem Strom zu Heizleistung verbessert Durch Einschalten des Induktors kann die Wärme in dem gesamten festen Heizmedium, das über eine sehr große Oberfläche mit dem Reaktionsgemisch Kontakt steht, erzeugt werden. Bei Abschalten des Induktors wird der weitere Wärmeeintrag sehr rasch unterbunden. Dies erlaubt eine sehr gezielte Reaktionsführung und minimiert die thermische Belastung des an der Reaktion beteiligten Übergangsmetall-Katalysators der vorliegenden Erfindung. Insbesondere thermisch labile Übergangsmetall-Katalysatoren behalten auf diese Weise über einen längeren Zeitraum hinweg ihre katalytische Aktivität, wodurch vergleichsweise höhere Reaktionsausbeuten in der Umesterungsreaktion der vorliegenden Erfindung erzielt werden können.

Das Heizmedium besteht aus einem elektrisch leitfähigen Material, das sich bei Einwirken eines elektromagnetischen Wechselfelds erwärmt. Es ist vorzugsweise ausgewählt aus Materialien, die im Vergleich zu ihrem Volumen eine sehr große Oberfläche aufweisen. Beispielsweise kann das Heizmedium ausgewählt sein aus jeweils elektrisch leitfähigen Spänen, Drähten, Netzen, Wolle, Membranen, porösen Fritten, Füllkörper wie beispielsweise Granulat, Raschig-Ringe und insbesondere aus Partikeln, die vorzugsweise einen mittleren Durchmesser von nicht mehr als 1 mm aufweisen. Um durch elektromagnetische Induktion erwärmbar zu sein, ist das Heizmedium elektrisch leitfähig, beispielsweise metallisch (wobei es diamagnetisch sein kann,) oder es zeigt eine gegenüber Diamagnetismus verstärkte Wechselwirkung mit einem Magnetfeld und ist insbesondere ferromagnetisch, ferrimagnetisch, paramagnetisch oder superparamagnetisch. Dabei ist es unerheblich, ob das Heizmedium organischer oder anorganischer Natur ist oder ob es sowohl anorganische als auch organische Komponenten enthält.

In einer bevorzugten Ausführungsform ist das Heizmedium ausgewählt aus Partikeln elektrisch leitfähiger und/oder magnetisierbarer Festkörper, wobei die Partikel eine mittlere Teilchengröße im Bereich von 1 bis 1000, insbesondere von 10 bis 500 nm haben. Die mittlere Teilchengröße und bei Bedarf auch die Teilchengrößenverteilung ist beispielsweise durch Lichtstreuung bestimmbar. Vorzugsweise wählt man magnetische Partikel, beispielsweise ferromagnetische oder superparamagnetische Partikel, die eine möglichst geringe Remanenz bzw. Restmagnetisierung aufweisen. Dies hat den Vorteil, dass die Partikel nicht aneinander haften. Die magnetischen Partikel können beispielsweise in Form sogenannter "Ferrofluide" vorliegen, also Flüssigkeiten, in denen ferromagnetische Partikel im nano-Größenmaßstab dispergiert sind. Die flüssige Phase des Ferrofluids kann dann als Reaktionsgemisch dienen.

Magnetisierbare Partikel, insbesondere ferromagnetische Partikel, welche die gewünschten Eigenschaften aufweisen, sind im Stand der Technik bekannt und kommerziell erhältlich. Beispielsweise seien die kommerziell erhältlichen Ferrofluide genannt. Beispiele für die Herstellung magnetischer nano-Partikel, die im Rahmen des erfindungsgemäßen Verfahrens verwendet werden können, können dem Aufsatz von Lu, Salabas und Schüth: "Magnetische nano-Partikel: Synthese, Stabilisierung, Funktionalisierung und Anwendung", Angew. Chem. 2007, 119, Seiten 1242 bis 1266 entnommen werden.

Geeignete magnetische nano-Partikel sind mit unterschiedlichen Zusammensetzungen und Phasen bekannt. Beispielsweise seien genannt: reine Metalle wie Fe, Co und Ni, Oxide wie Fe₃O₄ und gamma-Fe₂O₃, spinellartige Ferromagnete wie MgFe₂O₄, MnFe₂O₄ und CoFe₂O₄ sowie Legierungen wie CoPt₃ und FePt. Die magnetischen nano-Partikel können homogen aufgebaut sein oder eine Kern-Schale-Struktur besitzen. In letzterem Fall können Kern und Schale aus unterschiedlichen ferromagnetischen oder auch antiferromagnetischen Materialien bestehen. Es sind jedoch auch Ausführungsformen möglich, bei denen ein magnetisierbarer Kern, der beispielsweise ferromagnetisch, antiferromagnetisch, paramagnetisch oder superparamagnetisch sein kann, von einem nicht magnetischen Material umgeben ist. Dieses Material kann beispielsweise ein organisches Polymer darstellen. Durch eine solche Beschichtung kann eine chemische Wechselwirkung des Reaktionsgemisches mit dem Material der magnetischen Partikel selbst verhindert werden. Weiterhin kann das Material der Schale oberflächlich funktionalisiert werden, ohne dass das Material des magnetisierbaren Kerns in Wechselwirkung mit der funktionalisierenden Spezies tritt.

Als Heizmedium können beispielsweise nanoskalige Teilchen aus superparamagnetischen Stoffen eingesetzt werden, die ausgewählt sind aus Aluminium, Cobalt, Eisen, Nickel oder deren Legierungen, Metalloxiden vom Typ des n-Maghemits (gamma-Fe₂O₃), n-Magnetits (Fe₃O₄) oder der Ferritte vom Typ des MeFe₂O₄, wobei Me ein zweiwertiges Metall ausgewählt aus Mangan, Kupfer, Zink, Cobalt, Nickel, Magnesium, Calcium oder Cadmium ist. Vorzugsweise haben diese Teilchen eine durchschnittliche Teilchengröße von ≤ 100 nm, vorzugsweise ≤ = 51 nm und insbesondere bevorzugt ≤ 30 nm.

Als Heizmedium sind nanoskalige Ferrite einsetzbar, wie sie beispielsweise aus der WO 03/054102 bekannt sind. Diese Ferrite weisen eine Zusammensetzung (M^{a}_{1-x-y} M^{b}ₓ Fe^{II}_{y}) Fe^{III}₂O₄ auf, bei der
M^{a} ausgewählt ist aus Mn, Co, Ni, Mg, Ca, Cu, Zn, Y und V,
M^{b} ausgewählt ist aus Zn und Cd,
x für 0,05 bis 0,95, bevorzugt 0,01 bis 0,8 steht,
y für 0 bis 0,95 steht und
die Summe aus x und y höchstens 1 beträgt.

Die durch elektromagnetische Induktion erwärmbaren Partikel können ohne weitere Zusatzstoffe das Heizmedium darstellen. Es ist jedoch auch möglich, die durch elektromagnetische Induktion erwärmbaren Partikel mit anderen Partikeln zu mischen, die nicht durch elektromagnetische Induktion erwärmbar sind. Beispielsweise kann es sich hierbei um Sand handeln. Die induktiv erwärmbaren Partikel können also durch nicht induktiv erwärmbare Partikel verdünnt werden. Hierdurch kann eine verbesserte Temperaturkontrolle erreicht werden.

Werden nanoskalige durch elektromagnetische Induktion erwärmbare Partikel mit gröberen nicht induktiv erwärmbaren Partikeln vermischt, kann dies zu einer Verringerung der Packungsdichte des Heizmediums führen. Bei Ausführungsformen, bei denen das Reaktionsgemisch eine Packung aus dem Heizmedium durchströmt, kann dies eine erwünschte Reduktion des Druckverlusts im durchströmten Reaktor zur Folge haben.

Das feste Heizmedium kann oberflächlich mit einer katalytisch wirkenden Substanz belegt sein. Beispielsweise kann ein Übergangsmetallalkoxid der allgemeinen Formel (IV) auf der Oberfläche des Heizmediums immobilisiert werden, wodurch ebenfalls ein Übergangsmetall-Katalysator im Sinne der vorliegenden Erfindung erhalten wird.

In einer speziellen Ausführungsform der Erfindung trägt die Oberfläche des Heizmediums daher eine Hydroxylgruppen-haltige Schicht, die zur Anbindung, von Übergangsmetallalkoxiden der allgemeinen Formel (IV) verwendet werden kann. Alternativ dazu können die Übergangsmetallalkoxide der allgemeinen Formel (IV) vor und/oder während der Anbindung an die Hydroxylgruppen-haltige Schicht durch Teilhydrolyse in ein Übergangsmetalloligomer überführt werden, wobei der Begriff des "Übergangsmetalloligomers" wie oben definiert ist.

Insbesondere sind nanoskalige Teilchen als Heizmedium bevorzugt, die an der Oberfläche eine SiO₂-Schicht als Hydroxylgruppen-haltige Schicht aufweisen, die zur kovalenten Anbindung der nicht-hydrolysierten oder teilhydrolysierten Übergangsmetallalkoxide verwendet werden kann.

Prinzipiell kann die Umesterungsreaktion (Schritt a des vorliegenden Verfahrens) kontinuierlich oder chargenweise durchgeführt werden. Führt man die Reaktion chargenweise durch, geht man vorzugsweise so vor, dass man während der Reaktion das Reaktionsgemisch und das induktiv erwärmte feste Heizmedium relativ zu einander bewegt. Beim Verwenden eines partikelförmigen Heizmediums kann dies insbesondere dadurch erfolgen, dass man das Reaktionsgemisch zusammen mit dem Heizmedium rührt oder das Heizmedium in dem Reaktionsgemisch verwirbelt. Verwendet man beispielsweise Netze oder Wolle aus einem fadenförmig ausgestalteten Heizmedium, kann beispielsweise der Reaktionsbehälter, der das Reaktionsgemisch und das Heizmedium enthält, geschüttelt werden.

Eine bevorzugte Ausführungsform einer chargenweise durchgeführten Umesterungsreaktion besteht darin, dass sich das Reaktionsgemisch zusammen mit Partikeln des Heizmediums in einem Reaktionsbehälter befindet und mit Hilfe eines im Reaktionsgemisch befindlichen Bewegungselements bewegt wird, wobei das Bewegungselement als Induktor eingerichtet ist, durch den die Partikel des Heizmediums durch elektromagnetische Induktion erwärmt werden. In dieser Ausführungsform befindet sich also der Induktor innerhalb des Reaktionsgemisches. Das Bewegungselement kann beispielsweise als Rührer oder als sich auf und ab bewegender Stempel ausgebildet sein.

Man kann zusätzlich vorsehen, dass der Reaktor während der chemischen Reaktion von außen gekühlt wird. Dies ist insbesondere bei Chargenbetrieb möglich, wenn, wie vorstehend angegeben, der Induktor in das Reaktionsgemisch eintaucht. Das Einspeisen des elektromagnetischen Wechselfelds in den Reaktor wird dann nicht durch die Kühleinrichtung behindert.

Eine Kühlung des Reaktors kann von innen über Kühlschlangen oder Wärmetauscher oder vorzugsweise von außen erfolgen. Zur Kühlung kann man beispielsweise ggf. vorgekühltes Wasser oder auch eine Kühlmischung einsetzen, deren Temperatur unterhalb von 0 °C liegt. Beispiele solcher Kühlmischungen sind Eis-Kochsalz-Gemische, Methanol / Trockeneis oder flüssiger Stickstoff. Durch die Kühlung lässt sich ein Temperaturgradient zwischen der Reaktorwand und dem induktiv erwärmten Heizmedium herstellen. Dieser ist besonders ausgeprägt, wenn man eine Kühlmischung mit einer Temperatur deutlich unterhalb von 0 °C einsetzt, beispielsweise Methanol / Trockeneis oder flüssiger Stickstoff. Das Reaktionsgemisch, das durch das induktiv erwärmte Heizmedium aufgewärmt wird, wird dann wieder extern abgekühlt. Die Umesterungsreaktion der vorliegenden Erfindung findet somit vorzugsweise immer nur dann statt, wenn die Reaktanten Kontakt mit dem Heizmedium hat oder sich zumindest in dessen unmittelbarer Nähe befindet. Der der Reaktion entstandene Cyanacrylsäureester der allgemeinen Formel (I) gelangt durch die Relativbewegung des Reaktionsgemisches zum Heizmedium rasch in kühlere Bereiche des Reaktionsgemisches, so dass seine thermische Weiterreaktion gehemmt ist.

In einer alternativen Ausführungsform führt man die die Umesterungsreaktion der vorliegenden Erfindung kontinuierlich in einem Durchflussreaktor durch, der zumindest teilweise mit dem festen Heizmedium gefüllt ist und hierdurch mindestens eine durch elektromagnetische Induktion erwärmbare Heizzone aufweist, wobei das Reaktionsgemisch den Durchflussreaktor kontinuierlich durchströmt und wobei sich der Induktor außerhalb des Reaktors befindet. Vorzugsweise ist der Durchflussreaktor als Rohrreaktor ausgeführt. In diesem Fall kann der Induktor den Reaktor vollständig oder zumindest teilweise umgeben. Das vom Induktor erzeugte elektromagnetische Wechselfeld wird dann allseitig oder zumindest von mehreren Stellen aus in den Reaktor eingeleitet.

Bei dieser kontinuierlichen Verfahrensweise in einem Durchflussreaktor ist es möglich, dass der Reaktor mehrere Heizzonen aufweist. Beispielsweise können unterschiedliche Heizzonen unterschiedlich stark erwärmt werden. Dies kann entweder durch die Anordnung unterschiedlicher Heizmedien in dem Durchflussreaktor oder durch unterschiedlich ausgelegte Induktoren entlang des Reaktors erfolgen.

Weiterhin kann vorgesehen werden, dass das Reaktionsgemisch nach Verlassen der Heizzone mit einer Absorbersubstanz in Kontakt gebracht wird, die Nebenprodukte oder Verunreinigungen aus dem Reaktionsgemsich entfernt.

Je nach Reaktivität des Übergangsmetall-Katalysators und der verwendeten Reaktanten kann man die Produktausbeute ggf. dadurch erhöhen, dass das Reaktionsgemsich nach Durchströmen des festen Heizmediums zumindest teilweise zum erneuten Durchströmen des festen Heizmediums zurückgeführt wird. Dabei kann man nach dem jeweiligen Durchströmen des festen Heizmediums vorsehen, dass Verunreinigungen, Nebenprodukte oder auch das erwünschte Hauptprodukt aus dem Reaktionsgemisch entfernt werden. Hierfür sind die bekannten unterschiedlichen Abtrennverfahren geeignet, beispielsweise Absorption auf einer Absorbersubstanz, Ausfällen durch Kühlung oder destillative Abtrennung. Hierdurch kann letztlich eine vollständige Umsetzung der Reaktanten erreicht werden.

Die zweckmäßigerweise zu wählende gesamte Kontaktzeit des Reaktionsgemisches mit dem induktiv erwärmten Heizmedium hängt von der Kinetik der Umesterungsreaktion der vorliegenden Erfindung ab. Die Kontaktzeit ist um so länger zu wählen, je langsamer die jeweilige Umesterungsreaktion ist. Dies ist im Einzelfall empirisch anzupassen. Als Anhaltspunkt kann gelten, dass vorzugsweise das Reaktionsgemisch den Rohrreaktor mit einer solchen Geschwindigkeit einmal oder mehrmals durchströmt, dass die gesamte Kontaktzeit des Reaktionsgemisches mit dem induktiv erwärmten Heizmedium im Bereich von etwa 1 Sekunde bis etwa 2 Stunden liegt. Kürzere Kontaktzeiten sind denkbar, jedoch schwerer zu steuern. Längere Kontaktzeiten können bei besonders langsamen Umesterungsreaktionen der vorliegenden Erfindung erforderlich sein, verschlechtern jedoch zunehmend die Wirtschaftlichkeit des Verfahrens.

Unabhängig davon, ob man die Umesterungsreaktion der vorliegenden Erfindung chargenweise oder kontinuierlich in einem Durchflussreaktor durchführt, kann man vorsehen, dass der Reaktor als Druckreaktor ausgelegt ist und die Umesterungsreaktion bei einem Druck oberhalb von 1013 mbar, vorzugsweise von mindestens 1,5 bar durchgeführt wird.
In einer speziellen Ausführungsform des erfindungsgemäßen Verfahrens ist das Heizmedium ferromagnetisch und weist eine Curie-Temperatur im Bereich von etwa 40 bis etwa 250°C auf, die man so auswählt, dass sich die Curie-Temperatur um nicht mehr als 20°C, vorzugsweise nicht mehr als 10°C von der ausgewählten Reaktionstemperatur unterscheidet. Dies führt zu einem inhärenten Schutz vor einer unbeabsichtigten Überhitzung. Das Heizmedium lässt sich durch elektromagnetische Induktion nur bis zu seiner Curie-Temperatur aufheizen, während es bei einer darüber liegenden Temperatur nicht weiter durch das elektromagnetische Wechselfeld erwärmt wird. Selbst bei einer Fehlfunktion des Induktors kann auf diese Weise verhindert werden, dass die Temperatur des Reaktionsgemischs unbeabsichtigt auf einen Wert deutlich oberhalb der Curie-Temperatur des Heizmediums ansteigt. Fällt die Temperatur des Heizmediums wieder unter seine Curie-Temperatur ab, lässt es sich erneut durch elektromagnetische Induktion erwärmen. Dies führt zu einer Selbstregelung der Temperatur des Heizmediums im Bereich der Curie-Temperatur und erlaubt es auch thermisch labile Umesterungs-Katalysatoren mit einer hohen Verfahrenssicherheit einzusetzen, sofern deren Zerfallstemperatur unterhalb der Curie-Temperatur des Heizmediums liegt.

Es versteht sich von selbst, dass die Natur des Heizmediums und die Auslegung des Induktors so an einander angepasst werden müssen, dass sich die erwünschte Aufheizung des Reaktionsgemischs realisieren lässt. Eine kritische Größe hierfür ist einerseits die in Watt ausdrückbare Leistung des Induktors sowie die Frequenz des vom Induktor erzeugten Wechselfelds. Prinzipiell muss die Leistung umso höher gewählt werden, je größer die Masse des induktiv zu erwärmenden Heizmediums ist. In der Praxis ist die erzielbare Leistung insbesondere durch die Möglichkeit begrenzt, den zur Versorgung des Induktors erforderlichen Generator zu kühlen.

Besonders geeignet sind Induktoren, die ein Wechselfeld mit einer Frequenz im Bereich von etwa 1 bis etwa 100 kHz, insbesondere im Bereich von etwa 10 bis etwa 30 kHz erzeugen. Solche Induktoren sowie die zugehörigen Generatoren sind kommerziell erhältlich, beispielsweise von der IFF GmbH in Ismaning (Deutschland).

Nach Durchführung der Umesterungsreaktion (Schritt a) ist es vorteilhaft, den Übergangsmetall-Katalysator von der Reaktionsmischung abzutrennen. Die Abtrennung kann durch jede dem Fachmann geläufige Technik erfolgen, wobei insbesondere eine Abtrennung durch Filtration und/oder Zentrifugation bevorzugt ist.

Aufgrund der leichten Abtrennbarkeit des Übergangsmetall-Katalysators kann der im erfindungsgemäßem Verfahren erhaltene Cyanacrylsäureester der allgemeinen Formel (I) in hochreiner Form, insbesondere durch Destillation oder Kristallisation isoliert werden, wobei Produktreinheiten von größer 98% erzielt werden können.

Die so hergestellten Cyanacrylsäureester können zum Verkleben von Bauteilen, insbesondere zum Verkleben von elektrischen und/oder elektronischen Bauteilen sowie zur Herstellung von medizinischen Klebstoffen zur Behandlung von chirurgisch geschnittenem oder traumatisch gerissenem Gewebe verwendet werden.

Die vorliegende Erfindung wird, ohne sich auf diese zu beschränken, durch die nachfolgenden Beispiele näher erläutert

### Ausführungsbeispiele

### Verwendete Substanzen

Tetraisopropyltitanat und Methyllithium wurden von Sigma Aldrich bezogen und ohne weitere Aufreinigung eingesetzt. 1-Decanol wurde von Merck bezogen und ohne weitere Aufreinigung eingesetzt. Titantetrachlorid wurde und vor der Verwendung über Kupfer destilliert. Silicagel (Silica 60, 0.04 - 0.063 mm, 230 - 400 mesh, specific surface area 500 m²/g) wurde von Macherey-Nagel bezogen. Methyl-2-cyanacrylat [CAS 137-05-3] ist kommerziell erhältlich und kann vor dem Einsatz im erfindungsgemäßen Verfahren durch Destillation aufgereinigt werden.

### 1. Herstellung von Methyltrüsopropyltitanat

In einem 100 mL Schlenkkolben wurde zu Ti(O*i*-Pr)₄ (8,98 mL, 30,0 mmol) in trockenem Diethylether (5 mL) TiCl₄ (1,10 mL, 10,0 mmol) langsam bei 0°C zugegeben und die resultierende Mischung 10 min bei 0°C gerührt. Nach 30-minütigem Rühren bei 22°C wurde trockener Diethylether (15 mL) zugegeben und die Mischung auf -40 °C abgekühlt. Anschließend wurde eine Lösung von MeLi (1.6 M in Et₂O, 25,0 mL, 40.0 mmol) tropfenweise zugegeben. Die erhaltene gelbe Mischung wurde 10 min bei -40 °C gerührt und danach innerhalb von 45 min auf 0°C erwärmt. Durch Destillation unter reduziertem Druck konnte Methyltriisopropyltitanat (MeTi(O*i*-Pr)₃) isoliert werden.

### 2. Herstellung des Metallkatalysators 1

Eine Suspension von Silicagel (6,00 g, Vortrocknung im Vakuum bei 150°C für 20h) in wasserfreiem Toluol (30 mL) wurde mit einer Suspension von MeTi(O*i*-Pr)₃ (1,44 g, 6.00 mmol) in wasserfreiem Toluol (80 mL) versetzt. Die resultierende Reaktionsmischung wurde 16 h bei 22°C geschüttelt. Der erhaltene Metallkatalysator 1 wurde anschließend durch Filtration abgetrennt, mit Toluol gewaschen und für 24 h im Vakuum getrocknet.

### 3. Umesterungsreaktion

Ein beispielhafter Reaktionsaufbau kann der folgenden Publikation entnommen werden: Angew. Chem. 2008, 120, 9083 - 9086.

### Allgemeine Versuchsdurchführung

Ein Glasreaktor (14 cm Länge × 9 mm interner Durchmesser) wurde mit Stahlkugeln und dem Metallkatalysator 1 (1,8 g) befüllt. Nach Spülen des Reaktors mit wasserfreiem Toluol (10 mL, Flussrate: 0,5 mL/min) wurde eine Lösung von Methyl-2-cyanacrylat (MCA) (1,01 mL, 10,0 mmol, 10 eq.), 1-Decanol (0,19 mL, 1,00 mmol, 1 eq.) und Hydrochinon (0,005 eq./MCA) und Trifluormethansulfonsäure (0,001 eq./MCA) in wasserfreiem Toluol (10 mL) durch den Reaktor gepumpt (Flussrate 0,1 - 1,0 mL/min), wobei der Reaktor mittels einer induktiven Heizvorrichtung (Feldfrequenz 20 kHz, 310 Promille) auf ca. 90 °C erwärmt wurde. Anschließend wurde der Reaktor mit wasserfreiem Toluol (5 mL bei 0,2 mL/min, dann 10 mL bei 0,4 mL/min) gespült und die erhaltene Lösung im Vakuum konzentriert. Der Reaktoraustrag wurde mittels NMRspektroskopischer Methoden untersucht.

Durch das erfindungsgemäße Verfahren konnte Decyl-2-cyanacrylat in guten Ausbeuten isoliert werden, wobei das erhaltene Produkt nur einen geringen Anteil an Nebenprodukten aufwies.

## Patentansprüche

1. Verfahren zur Herstellung mindestens eines Cyanacrylsäureesters der allgemeinen Formel (I), 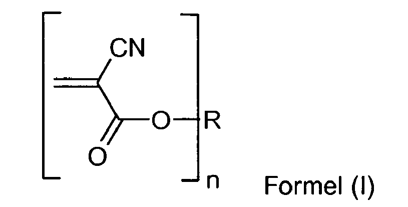 umfassend die Schritte:
a) Umesterung mindestens eines 2-Cyanacrylsäureesters der allgemeinen Formel (II), 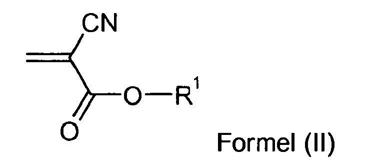 wobei R¹ ein verzweigter oder unverzweigter Alkylrest mit 1 bis 6 C-Atomen ist, mit mindestens einem Alkohol der allgemeinen Formel (III),
[H-O-]ₙR Formel (III)
in Gegenwart mindestens eines Übergangsmetall-Katalysators, der durch Umsetzung mindestens eines Hydroxylgruppen-haltigen Trägermaterials mit mindestens einem Übergangsmetallalkoxid gebildet wird und
b) Isolierung des in Schritt a) erhaltenen Cyanacrylsäureesters der allgemeinen Formel (I),
wobei n eine ganze Zahl von 1 bis 6 ist und R ein n-valenter Rest ist, der 1 bis 100 C-Atomen umfasst.

2. Verfahren nach Anspruch 1, **dadurch gekennzeichnet, dass** der Cyanacrylsäureester der allgemeinen Formel (I) ausgewählt wird aus Mono-cyanacrylsäureestern der allgemeinen Formel (la), 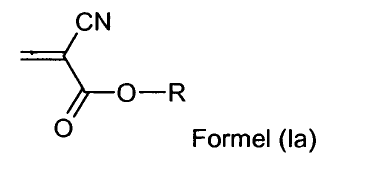 und/oder aus Biscyanacrylsäureestern der allgemeinen Formel (Ib), 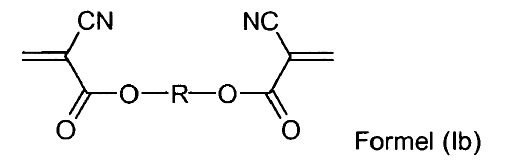 und/oder aus Triscyanacrylsäureestern der allgemeinen Formel (Ic), 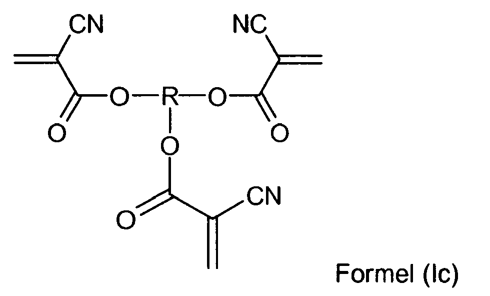 wobei der Rest R 1 bis 100 C-Atome umfasst.

3. Verfahren nach einem oder beiden der Ansprüche 1 und 2, **dadurch gekennzeichnet, dass** Rest R in Formel (I), Formel (la), Formel (Ib), Formel (Ic) und/oder Formel (III) eine C₃ bis C₁₀₀-Kette umfasst, die durch mindestens ein Sauerstoffatom unterbrochen ist.

4. Verfahren nach einem oder beiden der Ansprüche 1 und 2, **dadurch gekennzeichnet, dass** Rest R in Formel (I), Formel (la), Formel (Ib), Formel (Ic) und/oder Formel (III) 3 bis 18 direkt miteinander verbundene C-Atome umfasst.

5. Verfahren nach mindestens einem der Ansprüche 1 bis 4, **dadurch gekennzeichnet, dass** der 2-Cyanacrylsäureester der allgemeinen Formel (II) ausgewählt wird aus Methyl-2-cyanacrylat und Ethyl-2-cyansäureacrylat.

6. Verfahren nach mindestens einem der Ansprüche 1 bis 5, **dadurch gekennzeichnet, dass** das Hydroxylgruppen-haltige Trägermaterial ausgewählt wird aus Aluminiumoxid, Siliciumdioxid, Quarzstaub, Silicagel, Tonen, Zeolithen Zirconiumoxid, Titanoxid, Thoriumoxid oder Magnesiumoxid.

7. Verfahren nach mindestens einem der Ansprüche 1 bis 6, **dadurch gekennzeichnet, dass** das Übergangsmetallalkoxid ausgewählt wird aus Verbindungen der allgemeinen Formel (IV),
M(OR³)ₐ₋ᵤR⁴ᵤ Formel (IV)
wobei a für 3, 4 oder 5 steht, u entweder 0, 1 oder 2 ist, M für ein Übergangsmetall steht, R³ eine lineare oder verzweigte gegebenenfalls substituierte C1-20 Alkylgruppe oder eine gegebenenfalls substituierte C6-12 Arylgruppe ist und R⁴ für eine lineare oder verzweigte gegebenenfalls substituierte C1-4 Alkylgruppe steht

8. Verfahren nach mindestens einem der Ansprüche 1 bis 7, **dadurch gekennzeichnet, dass** mindestens ein Übergangsmetall im Übergangsmetallalkoxid ausgewählt wird aus Titan, Zirkonium, Hafnium, Eisen, Zink oder Vanadium.

9. Verfahren nach mindestens einem der Ansprüche 1 bis 8, **dadurch gekennzeichnet, dass** das Übergangsmetallalkoxid ausgewählt wird aus Titanalkoxiden.

10. Verfahren nach mindestens einem der Ansprüche 1 bis 9, **dadurch gekennzeichnet, dass** der Übergangsmetall-Katalysator erhalten wird durch Umsetzung mindestens eines Hydroxylgruppen-haltigen Trägermaterials mit mindestens einem Übergangsmetallalkoxid, wobei das Umsetzungsprodukt anschließend in Gegenwart von Sauerstoff Temperaturen von 150°C bis 800°C ausgesetzt wird.

11. Verfahren nach mindestens einem der Ansprüche 1 bis 10, **dadurch gekennzeichnet, dass** der mindestens eine Übergangsmetall-Katalysator in einer Menge von 0,01 bis 10 Gew.-%, bezogen auf die Gesamtmenge des 2-Cyanacrylsäureesters der allgemeinen Formel (II), vorliegt.

12. Verfahren nach mindestens einem der Ansprüche 1 bis 11, **dadurch gekennzeichnet, dass** das Verfahren in Gegenwart mindestens eines radikalischen Polymerisationsinhibitors und/oder mindestens einer gasförmigen Säure durchgeführt wird.

13. Verfahren nach mindestens einem der Ansprüche 1 bis 12, **dadurch gekennzeichnet, dass** die Umesterungsreaktion (Schritt a) unter Erwärmen eines Reaktionsgemisches in einem Reaktor in Gegenwart mindestens eines Übergangsmetall-Katalysators gemäß Anspruch 1 durchgeführt wird, wobei das Reaktionsgemisch mindestens einen Cyanacrylsäureester der allgemeinen Formel (II) und mindestens einen Alkohol der allgemeinen Formel (III) umfasst und in Kontakt mit einem durch elektromagnetische Induktion erwärmbaren festen Heizmedium steht, das sich innerhalb des Reaktors befindet und das durch elektromagnetische Induktion mit Hilfe eines Induktors erwärmt wird.

14. Verfahren nach Anspruch 13, **dadurch gekennzeichnet, dass** das Heizmedium ausgewählt ist aus Spänen, Drähten, Netzen, Wolle, Membranen, Fritten, Füllkörper und Partikeln, vorzugsweise ausgewählt aus Partikeln elektrisch leitfähiger und/oder magnetisierbarer Festkörper, wobei die Partikel eine mittlere Teilchengröße im Bereich von 1 bis 1000 nm haben.

15. Verfahren nach Anspruch 14, **dadurch gekennzeichnet, dass** das Heizmedium ausgewählt ist aus Partikeln magnetisierbarer Festkörper, wobei jedes Partikel einen Kern aus einem magnetisierbarem Material enthält, der von einem nicht magnetischen Material umhüllt ist.

## Claims

1. A method for producing at least one cyanoacrylic acid ester of the general formula (I), 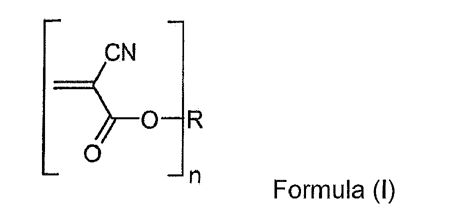 encompassing the following steps:
a) transesterification of at least one 2-cyanoacrylic acid ester of the general formula (II), 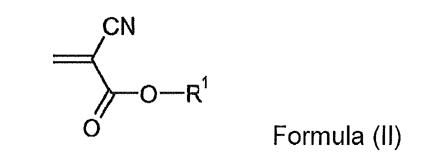 in which R¹ is a branched or unbranched alkyl residue having 1 to 6 C atoms, with at least one alcohol of the general formula (III),
[H-O-]ₙR Formula (III)
in the presence of at least one transition metal catalyst that is formed by reacting at least one hydroxyl group-containing support with at least one transition metal alkoxide, and
b) isolation of the cyanoacrylic acid ester of the general formula (I) obtained in step a),
n being a whole number from 1 to 6 and R being an n-valent residue encompassing 1 to 100 C atoms.

2. The method according to claim 1, **characterised in that** the cyanoacrylic acid ester of the general formula (I) is selected from monocyanoacrylic acid esters of the general formula (Ia), 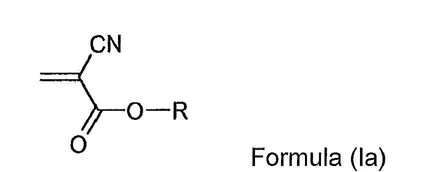 and/or from bis-cyanoacrylic acid esters of the general formula (Ib), 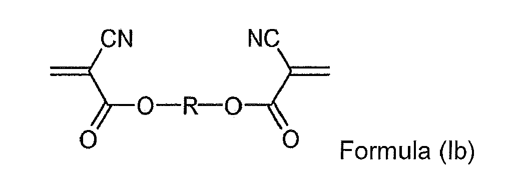 and/or from tris-cyanoacrylic acid esters of the general formula (Ic), 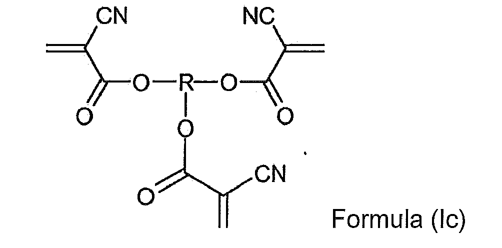 the residue R encompassing 1 to 100 C atoms.

3. The method according to one or both of claims 1 and 2, **characterised in that** the residue R in formula (I), formula (Ia), formula (Ib), formula (Ic) and/or formula (III) encompasses a C₃ to C₁₀₀ chain that is interrupted by at least one oxygen atom.

4. The method according to one or both of claims 1 and 2, **characterised in that** the residue R in formula (I), formula (Ia), formula (Ib), formula (Ic) and/or formula (III) encompasses 3 to 18 directly linked C atoms.

5. The method according to at least one of claims 1 to 4, **characterised in that** the 2-cyanoacrylic acid ester of the general formula (II) is selected from methyl-2-cyanoacrylate and ethyl-2-cyanoacrylic acid.

6. The method according to at least one of claims 1 to 5, **characterised in that** the hydroxyl group-containing support is selected from aluminum oxide, silicon dioxide, quartz dust, silica gel, clays, zeolites, zirconium oxide, titanium oxide, thorium oxide or magnesium oxide.

7. The method according to at least one of claims 1 to 6, **characterised in that** the transition metal alkoxide is selected from compounds of the general formula (IV),
M(OR³)ₐ₋ᵤR⁴ᵤ Formula (IV)
in which a denotes 3, 4 or 5, u is either 0, 1 or 2, M denotes a transition metal, R³ is a linear or branched optionally substituted C1-20 alkyl group or an optionally substituted C6-12 aryl group and R⁴ denotes a linear or branched optionally substituted C1-4 alkyl group.

8. The method according to at least one of claims 1 to 7, **characterised in that** at least one transition metal in the transition metal alkoxide is selected from titanium, zirconium, hafnium, iron, zinc or vanadium.

9. The method according to at least one of claims 1 to 8, **characterised in that** the transition metal alkoxide is selected from titanium alkoxides.

10. The method according to at least one of claims 1 to 9, **characterised in that** the transition metal catalyst is obtained by reacting at least one hydroxyl group-containing support with at least one transition metal alkoxide, the reaction product then being exposed to temperatures of 150°C to 800°C in the presence of oxygen.

11. The method according to at least one of claims 1 to 10, **characterised in that** the at least one transition metal catalyst is present in an amount from 0.01 to 10 wt.%, relative to the total amount of the 2-cyanoacrylic acid ester of the general formula (II).

12. The method according to at least one of claims 1 to 11, **characterised in that** the method is performed in the presence of at least one radical polymerization inhibitor and/or at least one gaseous acid.

13. The method according to at least one of claims 1 to 12, **characterised in that** the transesterification reaction (step a) is performed by heating a reaction mixture in a reactor in the presence of at least one transition metal catalyst according to claim 1, the reaction mixture encompassing at least one cyanoacrylic acid ester of the general formula (II) and at least one alcohol of the general formula (III) and being in contact with a solid heating medium that is capable of being heated by electromagnetic induction and that is located inside the reactor and is heated by electromagnetic induction with the aid of an inductor.

14. The method according to claim 13, **characterised in that** the heating medium is selected from chips, wires, meshes, wool, membranes, frits, fillers and particles, preferably selected from particles of electrically conductive and/or magnetizable solids, the particles having an average particle size in the range from 1 to 1000 nm.

15. The method according to claim 14, **characterised in that** the heating medium is selected from particles of magnetizable solids, each particle containing a core of a magnetizable material that is sheathed by a nonmagnetic material.

## Revendications

1. Procédé pour la préparation d'au moins un ester d'acide cyanacrylique répondant à la formule générale (I), 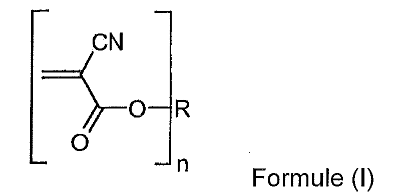 comprenant les étapes :
a) de transestérification d'au moins un ester d'acide 2-cyanacrylique répondant à la formule générale (II), 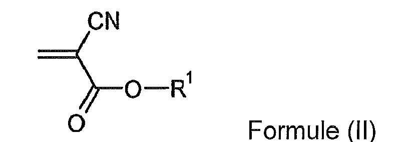 dans laquelle R¹ représente un résidu alkyle ramifié ou non ramifié contenant de 1 à 6 atomes de carbone, avec au moins un alcool répondant à la formule générale (III),
[H-O-]ₙR Formule (III)
en présence d'au moins un catalyseur à base d'un métal de transition que l'on obtient par mise en réaction d'au moins une matière de support contenant des groupes hydroxyle avec au moins un alcoxyde de métal de transition ; et
b) d'isolation de l'ester d'acide cyanacrylique que l'on obtient à l'étape a) répondant à la formule générale (I),
n représentant un nombre entier de 1 à 6 et R représentant un résidu à valence n qui comprend de 1 à 100 atomes de carbone.

2. Procédé selon la revendication 1, **caractérisé en ce que** l'ester d'acide cyanacrylique répondant à la formule générale (I) est choisi parmi des monoesters d'acide cyanacrylique répondant à la formule générale (la), 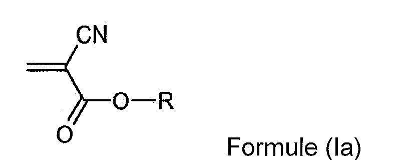 et/ou parmi des bis-esters d'acide cyanacrylique répondant à la formule générale (Ib), 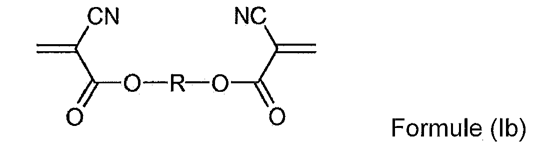 et/ou parmi des triesters d'acide cyanacrylique répondant à la formule générale (Ic), 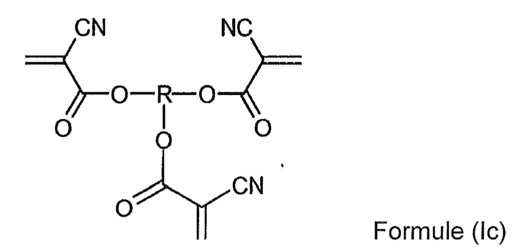 le résidu R comprenant de 1 à 100 atomes de carbone.

3. Procédé selon l'une quelconque des revendications 1 et 2 ou les deux, **caractérisé en ce que** le résidu R dans la formule (I), dans la formule (la), dans la formule (Ib), dans la formule (Ic) et/ou dans la formule (III) comprend une chaîne carbonée contenant de 3 à 100 atomes de carbone, qui est interrompue par au moins un atome d'oxygène.

4. Procédé selon l'une quelconque des revendications 1 et 2 ou les deux, **caractérisé en ce que** le résidu R dans la formule (I), dans la formule (la), dans la formule (Ib), dans la formule (Ic) et/ou dans la formule (III) comprend de 3 à 18 atomes de carbone liés directement les uns aux autres.

5. Procédé selon l'une quelconque des revendications 1 à 4, **caractérisé en ce que** l'ester de l'acide 2-cyanacrylique répondant à la formule générale (II) est choisi parmi le 2-cyanacrylate de méthyle et l'acrylate de l'acide éthyl-2-cyanique.

6. Procédé selon l'une quelconque des revendications 1 à 5, **caractérisé en ce que** la matière de support contenant des groupes hydroxyle est choisie parmi l'oxyde d'aluminium, le dioxyde de silicium, la poudre de quartz, le silicagel, des argiles, des zéolithes, l'oxyde de zirconium, l'oxyde de titane, l'oxyde de thorium ou l'oxyde de magnésium.

7. Procédé selon au moins une des revendications 1 à 6, **caractérisé en ce que** l'alcoxyde de métal de transition est choisi parmi des composés répondant à la formule générale (IV),
M(OR³)ₐ₋ᵤR⁴ᵤ Formule (IV)
dans laquelle a est égal à 3, 4 ou 5, u est égal à 0, 1 ou 2, M représente un métal de transition, R³ représente un groupe alkyle en C₁-C₂₀ linéaire ou ramifié, facultativement substitué ou un groupe aryle en C₆-C₁₂ facultativement substitué et R⁴ représente un groupe alkyle en C₁-C₄ linéaire ou ramifié, facultativement substitué.

8. Procédé selon au moins une des revendications 1 à 7, **caractérisé en ce qu'**au moins un métal de transition dans l'alcoxyde de métal de transition est choisi parmi le titane, le zirconium, le hafnium, le fer, le zinc ou le vanadium.

9. Procédé selon au moins une des revendications 1 à 8, **caractérisé en ce que** l'alcoxyde de métal de transition est choisi parmi des alcoxydes de titane.

10. Procédé selon au moins une des revendications 1 à 9, **caractérisé en ce que** l'on obtient le catalyseur à base d'un métal de transition par mise en réaction d'au moins une matière de support contenant des groupes hydroxyle avec au moins un alcoxyde de métal de transition, le produit réactionnel étant ensuite exposé en présence d'oxygène à des températures de 150°C à 800°C.

11. Procédé selon au moins une des revendications 1 à 10, **caractérisé en ce que** ledit au moins un catalyseur à base d'un métal de transition est présent en une quantité de 0,01 à 10 % en poids rapportés à la quantité totale de l'ester de l'acide 2-cyanacrylique répondant à la formule générale (II).

12. Procédé selon au moins une des revendications 1 à 11, **caractérisé en ce qu'**on met en oeuvre le procédé en présence d'au moins un inhibiteur de la polymérisation radicalaire et/ou d'au moins un acide formant un gaz.

13. Procédé selon au moins une des revendications 1 à 12, **caractérisé en ce qu'**on met en oeuvre la réaction de transestérification (étape a) par chauffage d'un mélange réactionnel dans un réacteur en présence d'au moins un catalyseur à base d'un métal de transition selon la revendication 1, le mélange réactionnel comprenant au moins un ester d'acide cyanacrylique répondant à la formule générale (II) et au moins un alcool répondant à la formule générale (III) et étant mis en contact avec un milieu de chauffage solide qui peut être chauffé par induction électromagnétique, qui se trouve à l'intérieur du réacteur et qui est chauffé par induction électromagnétique à l'aide d'un inducteur.

14. Procédé selon la revendication 13, **caractérisé en ce que** le milieu de chauffage est choisi parmi des copeaux, des fils, des filets, de la laine, des membranes, des frittes, des corps de remplissage et des particules, de préférence choisi parmi des particules de corps solides électroconducteurs et/ou magnétisables, les particules possédant une granulométrie moyenne dans la plage de 1 à 1000 nm.

15. Procédé selon la revendication 14, **caractérisé en ce que** le milieu de chauffage est choisi parmi des particules de corps solides magnétisables, chaque particule contenant un noyau constitué d'une matière magnétisable, qui est entouré d'un matériau amagnétique.
